(19) Europäisches Patentamt
European Patent Office
Office européen des brevets

(11) Publication number: **0 350 747 A2**

(12) **EUROPEAN PATENT APPLICATION**

(21) Application number: 89112087.5

(22) Date of filing: 03.07.89

(51) Int. Cl.[4]: C07D 311/58 , C08F 34/02

(30) Priority: 05.07.88 US 215149

(43) Date of publication of application: 17.01.90 Bulletin 90/03

(84) Designated Contracting States: AT BE CH DE ES FR GB IT LI NL SE

(71) Applicant: THE DOW CHEMICAL COMPANY
Office of Patent Counsel P.O. Box 1967
Midland Michigan 48640(US)

(72) Inventor: Inbasekaran, Muthiah
5800 Lamplighter
Midland Michigan 48640(US)
Inventor: Godschalx, James
2506 Longfellow Lane
Midland Michigan 48640(US)
Inventor: Dirlikov, Stoil K.
2430 Draper
Ypsilanti Michigan 48197(US)

(74) Representative: Sternagel, Hans-Günther, Dr. et al
Patentanwälte Dr. Michael Hann Dr. H.-G. Sternagel Sander Aue 30
D-5060 Bergisch Gladbach 2(DE)

(54) **Process for making and for polymerizing chromenes and products thereof.**

(57) This invention relates to a solvent process for the conversion of aromatic propargyl ethers into chromenes. The process can be catalyzed with copper or zinc salts. The preferred salt is cuprous chloride. The most preferred solvent is dichlorobenzene. The invention also relates to chromene products formed using the process and to the polymerization of that chromene product and its polymerized product, the latter product having substantially improved flexural modulus and flexural strength properties. The polymerized product is also moisture insensitive.

EP 0 350 747 A2

## PROCESS FOR MAKING AND FOR POLYMERIZING CHROMENES AND PRODUCTS THEREOF

The invention is related to the preparation and polymerization of chromenes which utilizes particular propargyl ethers. More particularly, the present invention provides a process for preparing chromenes by heating propargyl ethers in an appropriate solvent. These chromenes can then be polymerized. This invention also relates to the products formed from each of these processes. The polymers of this invention are utilized in the areas of high temperature composites and electrical laminates.

The invention relates to a solvent process for the preparation of thermosetting compositions containing polymerizable chromenes derived from aromatic propargyl ethers. The polymerized products have improved yields and unexpectedly result in substantial improvement in the flexural modulus and flexural strength compared to other state of the art thermoset resins.

U.S. Patent No. 4,226,800 to Picklesimer teaches polymerization of propargyl ethers to polymers.

It reports a process wherein a phenolic material is reacted with propargyl bromide in aqueous sodium hydroxide solution to afford propargyl ethers. The resultant ethers are then thermally polymerized without solvent to hard polymers. The reference does not disclose the intermediacy of chromenes in the polymerization process. Additionally, no data is given on the properties of the hard polymers to be able to assess whether the materials are useful.

In addition to the mentioned disadvantages, the process for preparing the propargyl ethers suffers from the further disadvantage of providing both C-alkylated (desired) and C-alkylated (undesired) materials. For example, bisphenol A is claimed to provide 45.4 percent yield of the desired bispropargyl ether and 43.6 percent yield of the undesired C-propargylated bisphenol. Additionally, the process uses rather vigorous conditions, such as reflux conditions of 100 °C from 1 to 3 hours. A further drawback of the process is that propargyl bromide is used rather than propargyl chloride. The bromide is relatively expensive, inaccessible on the commercial scale, and shock sensitive according to Fire Technology, 5,100 (1969).

IBM Technical Disclosure Bulletin, Vol. 27, No. 4B, pp. 25-29, (September, 1984), also describes direct polymerization of polypropargyl ethers to polymers without description of the properties and the quality of the resulting polymers or reference to any intermediates. Specifically, the bulletin describes that a mixture containing a 1:1 ratio by weight of aromatic propargyl ethers:

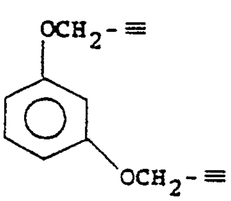

and

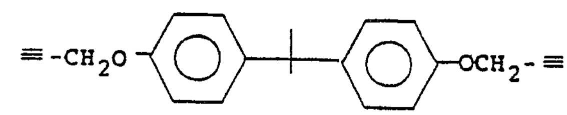

can be polymerized by heating at 130 °C for several minutes and then at 250 °C for at least an hour. The process described to prepare the propargyl ethers utilizes propargyl bromide and thus suffers from one of the same disadvantages as the preceding reference. In addition, not enough detail is given for one skilled in the art to reproduce the work.

M. Harfenist and E. Thom, The Influence Of Structure On The Rate Of Thermal Rearrangement Of Aryl Propargyl Ethers To The Chromenes, J. Org. Chem; Vol. 37, No. 6, p. 841 (1972), describe the cyclization of monofunctional propargyl ethers to monofunctional chromenes in yields of 36 to 76 percent by heating in a solvent such as dimethylaniline, trimethylene glycol, or dichlorobenzene. Yield loss is attributed to tar formation. The reference teaches that reheating isolated chromene products gave tar formation at a sufficiently fast rate to account for the deviation from quantitative yield in the cases studied. According to the authors, this indicated that the rate of loss of acetylenic hydrogen, which was measured in the kinetic runs, corresponded in all probability to formation of the chromene and that tar formation was a subsequent reaction of the chromene.

W. Anderson and E. LaVoie, Thermal Cyclization Of Substituted Aryl Propargyl Ethers. The Scope And Regioselectivity Of The Reaction In The Synthesis Of Substituted 3-Chromenes, J. Org. Chem., Vol. 38, No. 22, p. 3832 (1973), describe the thermal cyclization of substituted aryl propargyl ethers. There, it was reported that simple 3-aryloxypropynes were cyclized to the corresponding chromenes in approximately 60 percent yield. The solvent diethylaniline was used at between 210 to 215 °C.

K. Balasubramanian and B. Venugopalan, Studies in Claisen Rearrangements: Claisen Rearrangement

of Bispropargyl Ethers, Tetrahedron Letters, No. 29, p. 2707, (1973), reported the formation of bischromenes derived from the bispropargyl ethers of 2,7-dihydroxynaphthalene and hydroquinone by heating the latter compounds in N,N-diethylaniline.

S. Powell and R. Adams, A Comparison of the Activity of Certain Unsaturated Groups with the Activity of the Allyl Group in Certain Ethers, J. Chem. Soc., Vol. 42, p. 646 (1920), reported that phenyl and p-bromophenyl propargyl ethers decomposed upon heating with or without a solvent to tarry mixtures.

In addition to solvent systems for chromene formulation, catalytic processes are also known. According to Zsindely and Schmid, Sigmatropische Umlagerungen von Aryl-propargylathern; Synthese von 1, 5-Dimethyl-6-methylene-tricyclo [3,2,1,$0^{2,7}$]-oct-3-en-8-on Derivaten, Helv. Chim. Acta, 51, 1510 (1968), aromatic propargyl ethers rearrange at about 200 °C to chromenes. Later, Koch-Pomeranz, Hansen, and Schmid, Die Durch Silberionen Katalysierte Umlagerung von Propargyl-phenylather, Helv. Chim. Acta, 56, 2981 (1973) demonstrated that the rearrangement was catalyzed by silver tetrafluoroborate or silver trifluoroacetate. According to the authors, the presence of catalysts enabled the reaction to be carried out at lower temperatures (20 to 80 °C) using benzene or chloroform as solvent. There, the silver salts were used at 45 to 330 mol percent levels. Both of the preceding references are hereby incorporated by reference.

Balasubramanian, Reddy and Nagarajan, A Novel Mercuric Ion Catalysed Reaction of 1,6-Diaryloxy-2,4-Hexadiynes to 4-4′-Bichromenes, Tetrahedron Letters, 50, 5003 (1973), reported that the rearrangement of bispropargyl ether:

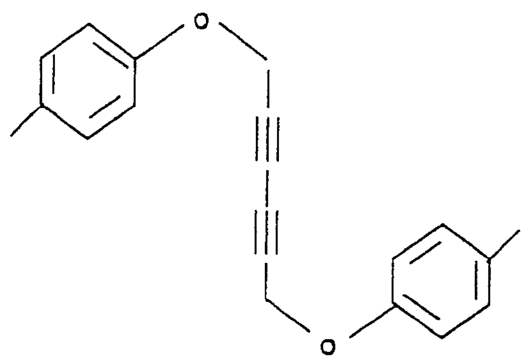

into the bischromene:

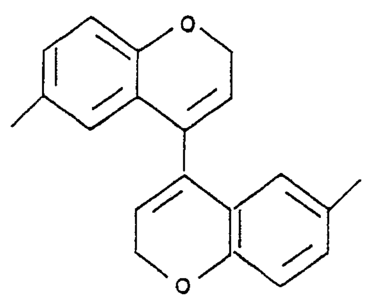

was catalyzed by mercuric oxide and concentrated sulfuric acid. The bischromene was obtained in 61 percent held as crude, impure material and had to be purified by column chromatography. The relative amount of the catalyst was not specified in the publication.

The present invention overcomes disadvantages taught in the prior art. For example, U.S. Patent No. 4,226,800 and the IBM Technical Disclosure Bulletin describe a direct polymerization process which is a highly exothermic process; the heat of polymerization is in excess of 1100 Joules per gram. Description of the properties, quality of the resulting polymers and their identification were not disclosed in the references. It is known that such high heats of polymerization lead to bubble formation weakening the resulting polymer product and thermal stresses lead to cracking of the polymer product.

In contrast, the polymerizable mixtures of applicants' invention are used to make substantially void-free moldings with minimal shrinkage. This can be attributed to mild heats of polymerization. The reduction in the heat of polymerization has been achieved by the Applicants through the partial conversion of the aromatic propargyl ether group into chromene functionality. Moreover, the polymers of the present invention

have excellent properties, for example, flexural modulus and flexural strength. In particular, applicants' product is substantially free of oligomeric or polymeric species which substantially increase the viscosity of the mixture. It has been discovered by the applicants that heating propargyl ethers without a solvent present leads to fairly rapid formation of the undesirable oligomeric or polymeric species.

Moreover, thermal rearrangement of aromatic propargyl ethers in the references leads to chromenes in yields of 36 to 76 percent. The yield loss was attributed to tar formation according to the article by Harfenist and Thom. The present invention does not require complete conversion of propargyl ethers to chromenes to give the desired product. Thus, the conversion can be stopped before undesirable tar formation occurs. Because no tar formation occurs and no volatiles are liberated during the conversion the mass balance of the applicants' invention is near quantitative. Additionally, applicants' catalyzed system leads to shorter reaction times for conversion of the propargyl ethers to chromenes than the processes described in the prior art. Regarding applicants' catalyst system, it is unnecessary for them to remove catalyst because small amounts of an inexpensive catalyst are utilized.

Reported catalysts of silver and mercury salts are disadvantageous. Silver salts are expensive, particularly in the concentrations used by Koch-Pomeranz et al. (45 to 3000 mole percent). Additionally, this reference teaches that undesirable benzofuran by-products are obtained in the silver tetrafluoroborate-catalyzed rearrangement. Use of mercury salts is undesirable due to their known toxicity and associated environmental concerns.

However, applicants have surprisingly discovered that copper and zinc salts effectively catalyze the conversion of propargyl ethers into chromenes while using relatively minor amounts.

With the disadvantages of the prior art and the advantages of applicants' process and products in mind, a more detailed description of applicants' invention follows.

This invention relates to a process for the preparation of chromene containing mixtures comprising:
reacting a material containing propargyl ether groups in a suitable solvent at reaction temperature; the material containing the propargyl ether groups being represented by the following formula I:

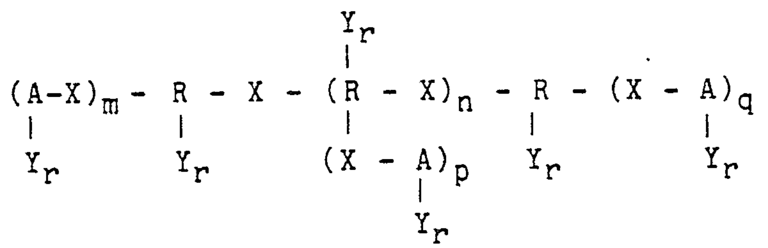

I

where n is an integer from 0 to 200; m, p, q are integers independently from 0 to 6; m + p + q is greater than or equal to 2; r is an integer independently from 0 to 10; A is a $C_6$-$C_{20}$ aromatic radical optionally substituted with Y and bearing at least one propargyl ether group provided that at least one of the positions ortho to the propargyl ether group be unsubstituted; R is a hydrocarbyl radical having from 0 to 50 carbon atoms optionally substituted with Y; and Y is halogen,
-OAr, $-OR'$, $-SR'$, $-SO_2Ar$, $-SO_2R'$, $-CO-R'$, -CO-Ar,
$O\overset{O}{\overset{\|}{C}}-R'$, $-O-\overset{O}{\overset{\|}{C}}-Ar$, $NO_2$, $-NH\overset{O}{\overset{\|}{C}}R'$, $-NH\overset{O}{\overset{\|}{C}}Ar$, $-\overset{O}{\overset{\|}{C}}-NHR'$, $-\overset{O}{\overset{\|}{C}}-NHAr$, $-SiR'_3$, $-Si(OR')_3$, $R'$, H, -OH, or $-CO_2R'$;
$R'$ is a hydrocarbyl radical having from 0 to 20 carbon atoms; x is absent or is:

$$-\underset{R}{\overset{R'}{C}}-,\ -\underset{CF_3}{\overset{CF_3}{C}}-,\ -SO_2-,\ -CO-,\ -O-,\ -S-,\ -SO-,\ -\underset{R'}{\overset{O}{\overset{\|}{P}}}-,$$

$$-O-\underset{O}{\overset{O}{\overset{\|}{P}}}-O,\ -O-\overset{O}{\overset{\|}{P}}-O-,\ -\underset{R}{\overset{OR'}{P}}-,\ -\overset{O}{\overset{\|}{C}}-O-,\ -O-\overset{O}{\overset{\|}{C}}-O,\ -\overset{O}{\overset{\|}{C}}-NH-,\ -\overset{O}{\overset{\|}{C}}-NR'-,$$

$$-\overset{O}{\overset{\|}{C}}-NAr-,\ \text{or}\ -NH-\overset{O}{\overset{\|}{C}}-NH-;$$

preferably

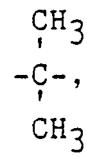 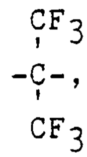 $-SO_2-$, or $-CO-$;

and recovering chromene containing mixtures having the following formula II:

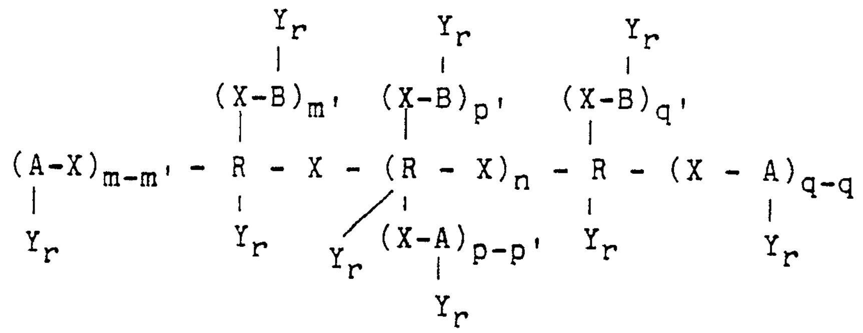

II

where R, Y, X n, m, p, q, r, A have the representations recited above; m′, p′, q′ are integers from 0 to 6; m′ + p′ + q′ is greater than or equal to 0; and B is a $C_6$-$C_{20}$ aromatic radical containing at least one chromene group.

Preferably, the reaction is carried out in a solvent of diethylbenzene, diisopropylbenzene, dichlorobenzene, trichlorobenzene or tetralin at a reaction temperature between 100 to 200° C. More preferably, the reaction is carried out in the presence of a catalyst of copper or zinc halide or acetate in an amount between 10 to 10,000 ppm, more preferably 50 to 500 ppm.

This invention also relates to an in situ process for the formation of chromene containing mixtures comprising:

reacting a phenolic compound with propargyl halide in an aqueous solution of an alkaline agent in the presence of a phase transfer catalyst under reaction conditions sufficient to produce a propargyl ether;

adding an inert organic solvent to the propargyl ether;

purifying the solubilized propargyl ether;

reacting the propargyl ether is a suitable solvent at reaction temperature; the material containing the propargyl ether groups being represented by formula 1; and

recovering a chromene containing mixture having formula II.

Phenolic compounds used in the practice of this invention are aromatic or alkyl aromatic compounds which bear one or more hydroxyl moieties. These compounds are generally represented by the following formulas:

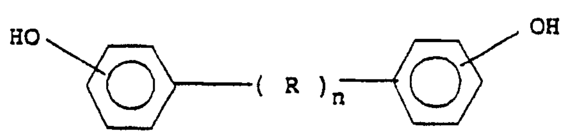

A

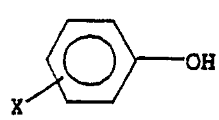

B

wherein R is independently O, S, CO, $SO_2$,So, $C(CH_3)_2$ or $C(CF_3)_2$ and x is 4-OPh, 3-Me or 4-Me, and n is 0 or 1. Preferred hydroxy aromatic reactants are those aromatic compounds for Formula A wherein the 2 hydroxyl moieties are in the 4 and 4′ positions and further wherein n is 1. If R is a divalent hydrocarbon

radical, it will have from 1 to about 4 carbon atoms. The most preferred hydroxy aromatic reactant is bisphenol A wherein R is $C(CH_3)_2$. The hydroxy aromatic reactants may bear groups or substituents which do not interfere with the alkylation reaction of this invention.

Propargyl halides suitably employed in the practice of this invention are represented by the following formula C: $HC \equiv C\text{-}CH_2\text{-}Y$ C
wherein Y is chlorine, bromine or iodine. Preferred propargyl halides are propargyl chloride and propargyl bromide. The most preferred propargyl halide is propargyl chloride. For every one mole equivalent of phenolic material, the present invention employs 1 to 1.5 mole equivalents of the propargyl halide, preferably 1 to 1.15 mole equivalents of the propargyl halide. An alkaline agent is used in the practice of the present invention for the purpose of increasing conversion to the propargyl ether. For example, the alkaline agent may be an alkaline metal hydroxide or an alkaline earth metal hydroxide. Preferred alkaline agents are potassium hydroxide, sodium hydroxide or mixtures thereof. Sodium hydroxide is the most preferred alkaline agent. For every 1 mole equivalent of the phenol, 1 to 10 mole equivalents of caustic solution are used, preferably 2 to 4 mole equivalents of caustic solution.

A phase transfer catalyst is used in the process of the present invention. It is used for the purpose of providing unexpected yields of propargyl ether product at higher conversion of the hydroxy aromatic reactants to the desired product. The purity of the product resulting from selectivity of the catalyst are unexpected. In many cases, the catalyst speeds the rate of reaction and improves and speeds the dissolution of transient phenate salts. Suitable catalyst are quaternary salts, poly(ethylene glycol), poly-(ethylene glycol alkylether), crown ethers, or cryptates. Quaternary salts are represented by the formula D.

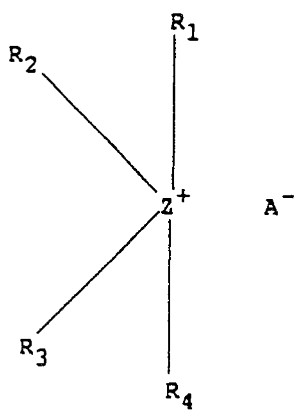

D

wherein Z is a tetravalent ammonium or phosphonium or arsonium ion, A is any suitable counter ion, and each $R_1$, $R_2$, $R_3$ and $R_4$ is independently an alkyl, aromatic or alkyaromatic moiety containing from 1 to 50 carbon atoms, preferably 1 to 5 carbon atoms or a benzyl radical. The catalyst is preferably employed in an amount which provides a ratio of the catalyst to the reactants of from 1:100 to 10:100, more preferably, from 2:100 to 5:100.

This invention further relates to a polymer comprising the compound of formula I, an electrical laminate comprising thereof, a chromene containing mixture comprising the compound of formula II, and a process for the polymerization of the chromene containing mixture. The chromene containing mixtures may exclude those chromene based on 2,7 dihydroxynaphthalene and hydroquinone.

Commercially available high temperature thermosetting resins are moisture sensitive and are difficult to process. This invention overcomes these problems through the discovery of a new process, a chromene containing product, and a polymerization product thereof. This is achieved by converting a propargyl ether directly to a low viscosity liquid containing chromenes which are more easily processed. This chromene containing product is polymerized. The cured polymer shows excellent electrical and mechanical properties up to 232 °C (450 °F). This polymer is moisture insensitive and can be used in the filed of high temperature composites and electrical laminates. The several embodiments of this invention will be described in greater detail below.

## Propargyl Ether Formation

The aromatic propargyl ether is made according to the teachings set forth in U.S. Patent Application

Serial No. 056,190, filed June 1, 1987, to Inbasekaran et al, hereby incorporated by reference. That process involves stirring phenolic material and the propargyl halide in aqueous caustic solution in the presence of a phase transfer catalyst. The phase transfer catalyst can be tetraalkylated ammonium halides, tetraalkylated phosphonium halides or tetraalkyl arsonium halides having an alkyl radical of 1 to 50 carbon atoms or a benzyl group. The halide can be iodide, bromide, chloride or fluoride. The phase transfer catalyst can also be poly(ethylene glycol) (molecular weight equals 200 to 50,000) or poly(ethylene glycol alkyl ether) (molecular weight equals 200 to 50,000), crown ethers or cryptates. The caustic solution can be either a solution of sodium hydroxide or potassium hydroxide. The phenolic material is preferably a bisphenolic compound having two aromatic rings. Operating temperatures during ether synthesis range from 0° to 100° C, preferably 200 to 50° C.

Formation of Chromenes Directly From Bisphenol A

In a further embodiment of the invention, chromenes can be formed in a single step from bisphenol A and propargyl chloride without isolating aromatic propargyl ethers. This eliminates steps and makes the process more economical. This single step process also reduces waste levels.

Polymerization of Chromenes

Another embodiment of the invention relates to the polymeric composition III derived by heating the chromene containing composition II, which, in the basic situation is depicted as:

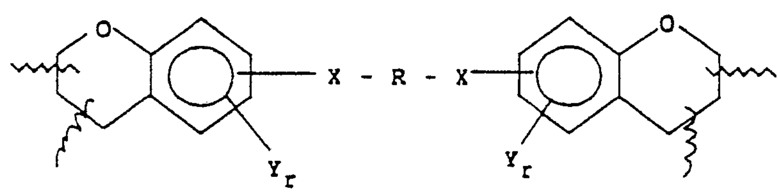

III

More specifically, the invention relates to the chromene IV and V derived from bisphenol A bispropargyl ether VI and the polymers derived from IV, V and VI.

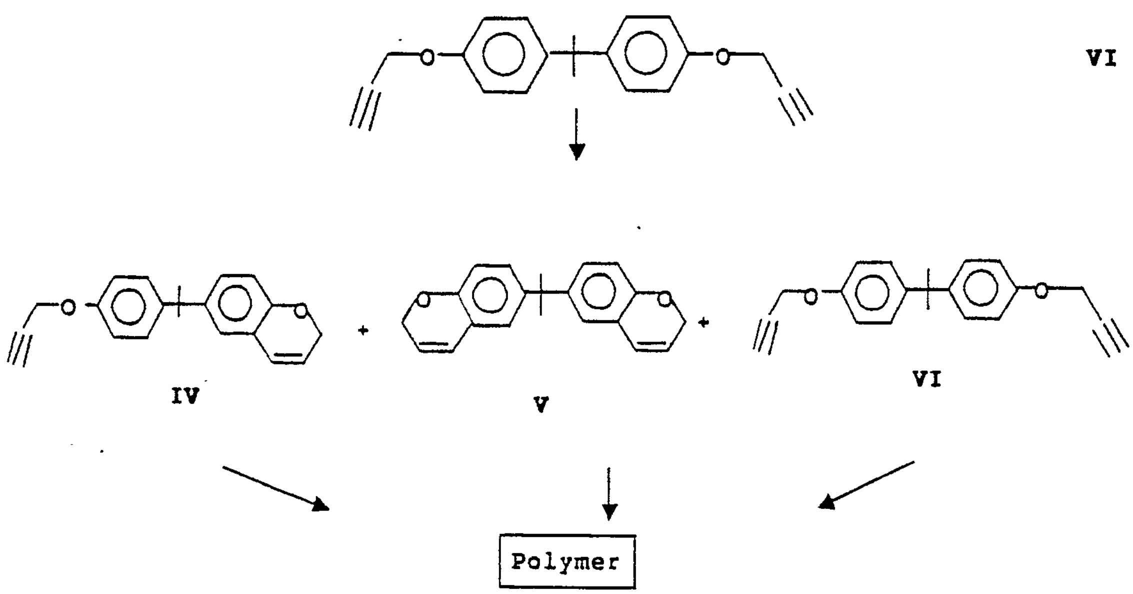


VI

IV + V + VI

Each of these embodiments will be described in greater detail below.

Formation of Chromenes From Aromatic Propargyl Ethers

According to the present invention, an aromatic propargyl ether such as I is heated in a solvent at 100° C to 220° C for a period of time ranging from 2 to 100 hours, more preferably 2 to 12 hours, under subatmospheric, atmospheric, or superatmospheric pressure. Any suitable solvent can be used. Depending on the boiling point of the solvent, the desired reaction temperature can be maintained by adjusting the pressure under which the reaction is conducted. If is preferred to conduct the reaction under atmospheric pressure and in the temperature range of 100° to 200° C, more preferably, 150° to 185° C. Therefore, the preferred solvents are those with boiling points in the same temperature range. Specifically, diethylbenzene, diisopropylbenzene, dichlorobenzene, trichlorobenzene, and tetralin are preferred solvents. The choice of reaction conditions, that is reaction temperature and time, is determined by the extent of conversion of aromatic propargyl ether I that is desired. A longer reaction, or higher reaction temperature will provide a greater conversion. Other reaction conditions are conventional. Removal of the solvent by conventional techniques, for example, distillation at reduced pressures, provides the product in excellent yield.

The desired product, represented by structure II is a mixture comprising compounds where $m' + p' + q'$ independently varies from 0 to $m + p + q$. For instance, when $m + p + q$ equals 2, then the product will be a mixture of unreacted ether I, the dichromene compound, and the monoproparglу ether-monochromene compound. Because of the large number of compounds possible when $m + p + q$ is larger than 2, it is convenient to characterized the conversion and the product mixture by the percentage of chromene moiety in the product mixture. Thus, where $m + p + q$ equals 2, a 50 percent chromene-containing product will be an equimolar mixture of unreacted ether I, the dichromene compound, and the monopropargyl ether-monochromene compound. The composition of the product of this invention can vary from 10 percent to 100 percent chromene. The preferred composition is 30 to 75 percent chromene, and the most preferred composition is 50 to 70 percent chromene. The selection of composition is determined by the ease of subsequent polymerization of the chromene mixture and the quality of the resulting polymer. The conversion of propargyl ethers to chromenes can be monitored by gas chromatography which provides information about composition of the product. Because the conversion can be stopped when desired, a desired product mixture can be obtained. The desired product mixture can also be obtained by blending a mixture of large chromene content with the appropriate amounts of an aromatic propargyl ether. Percentages are by weight of the composition.

Catalytic Process

According to another feature of the present invention, an aromatic propargyl ether I is heated in a solvent at 100° to 200° C, more preferably at 150° to 185° C in the presence of 10 to 10,000 ppm, more preferably 50 to 500 ppm, of a copper or zinc salt for a period of time ranging from 1 to 1,000 hours, more preferably 2 to 12 hours, under subatmospheric, atmospheric or superatmospheric pressure. The catalytic conversion of the bispropargyl ether of bisphenol A (VI) into the mixture of ether VI and chromenes IV and V can be accomplished in substantially less time using 100 ppm copper (I) chloride as catalyst than without catalyst present.

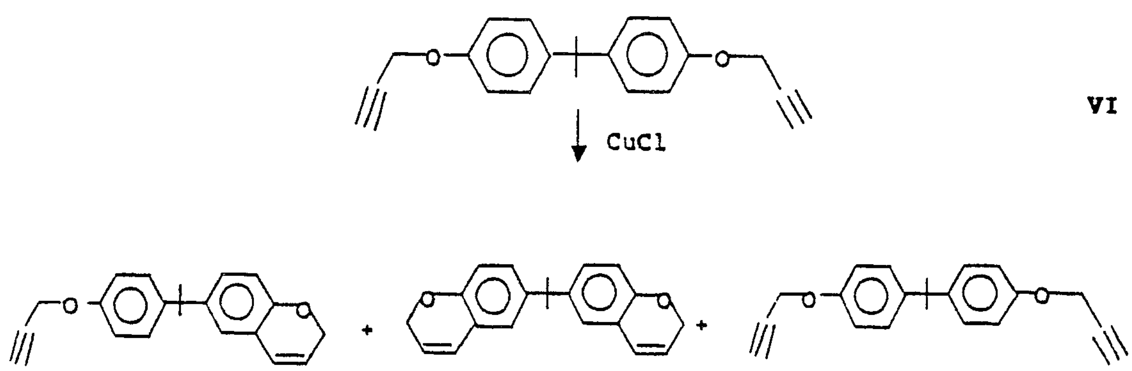


VI

IV + V + VI

Any suitable solvent can be used. Again, the preferred solvents are dichlorobenzene, trichlorobenzene,

diethylbenzene, diisopropylbenzene and tetralin. The most preferred solvent is dichlorobenzene. Any copper (I), copper (II) or zinc (II) salt can be used, but the preferred salts are the halides, acetates, bromides, chlorides, cyanides, fluorides, nitrates, phosphates, sulfates, alkoxides, trifluoroacetates, methanesulfonates, benzenesulfonates, p-toluene sulfonates, tetrafluoroborate, hexafluoroborates, acetylacetonates, gluconates, etc. The most preferred catalyst is cuprous chloride conveniently used as a solution in concentrated hydrochloric acid. The concentration of the catalyst is from about 10 to 10,000 ppm (0.001 to 1 weight percent), preferably from 50 to 500 ppm (0.005 to 0.05 weight percent).

Formation of Chromenes Directly From Bisphenol A

In another embodiment of the present invention, the single-step process involves stirring phenolic material and two molar equivalents of propargyl chloride in aqueous caustic solution in the presence of a phase transfer catalyst. The catalyst can be tetraalkylammonium halides, tetraalkylphosphonium halides, poly(ethylene glycol), poly(ethylene glycol alkyl ether), crown ethers or cryptates. Operating temperature ranges are from 0 to 100° C, preferably 20 to 50° C, most preferably 40 to 45° C. The preferred catalysts are the tetrabutylammonium bromide, tetrabutylphosphonium bromide and the corresponding iodide salts. The most preferred catalyst is tetrabutylammonium bromide.

The preferred ratio of the catalyst to the substrate is 1:100 to 10:100, preferred range being 2:100 to 5:100. The reaction time depends upon operating temperature. At 45° C, the reaction takes 2 hours to consume most of the starting materials. It is preferred to add an excess of 10 mole percent of propargyl chloride after 2 hours, and permit the reaction to proceed to completion at 45° C for another two hours.

The next phase of this single-step process involves addition of an inert organic solvent to selectively solubilize the propargyl ether. As explained previously, solvents can have a wide range of boiling points between 100 to 220° C with a preferred range being 150 to 220° C. The preferred solvents are diethylbenzene, diisopropylbenzene, dichloro- and trichlorobenzene, decalin and tetralin. The most preferred solvent is orthodichlorobenzene. The solution of propargyl ether I in the added solvent separates from the aqueous layer.

The third phase of the present process involves purification of the solution of propargyl ether I. Small amounts of undesirable basic impurities such as sodium phenates are effectively removed by washing with a dilute aqueous solution of a mineral acid such as hydrochloric, sulfuric or nitric acid or a carboxylic acid such as acetic acid or by passing through a column of cation exchange resin such as DOWEX MSC-1 (Trademark of The Dow Chemical Company). The latter process is preferred over the former because the ion exchange resin also acts as a desiccant and removes water effectively from the organic solution of propargyl ether I. The cation exchange resin is washed with small amounts of an inert solvent such as toluene, benzene, chlorobenzene, dichlorobenzene, and the like to free any residual propargyl ether. Gas chomatographic analysis of the organic solution shows the purity of the desired propargyl ether I to be 97 to 98 percent.

Conversion of the solution of propargyl ether I into chromene-acetylene mixture II is carried out as taught above. The most preferred catalyst for the conversion of propargyl ether I into chromene-acetylene mixture II is a solution of cuprous chloride in concentrated hydrochloric acid. Fifty (50) to 100 ppm of the catalyst is added. The solution is heated to reflux with the collection of volatile materials (for example, propargyl chloride used in excess, toluene used for washing, etc.). The reflux is carried out for 1 to 24 hours, preferably 2 to 8 hours and most preferably 3 to 5 hours. Progress of the conversion is monitored by gas chromatography. When the most preferred composition of the chromene-acetylene mixture containing 50 to 70 percent chromenes is achieved, the reaction is stopped. The solvent is removed under reduced pressure. The chromene-acetylene mixture is obtained in 90 to 95 percent yield based on the starting phenolic material.

The process is depicted by the preparation of the chromene-acetylene resin containing the species, VI, IV and V starting from bisphenol A and propargyl chloride:

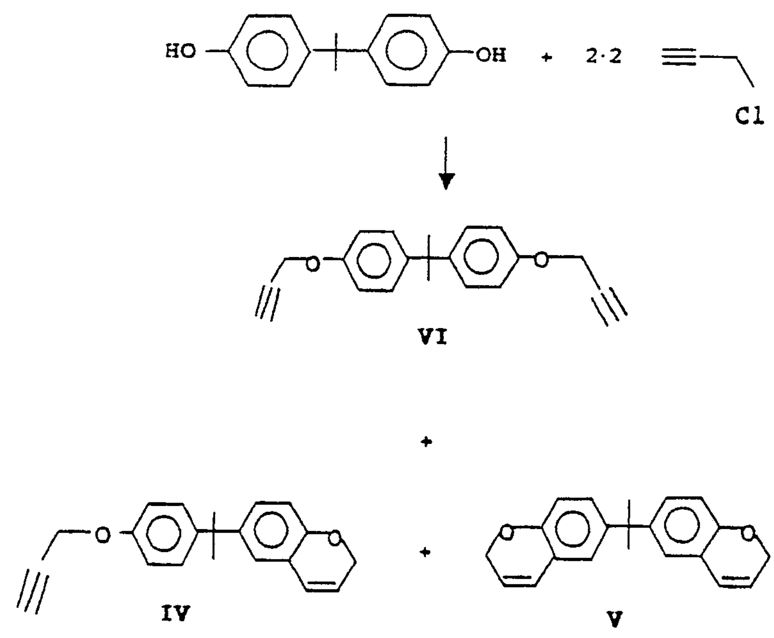

VI

IV

V

## Polymerization of Chromenes

The polymerization of the chromene-containing compounds is typically conducted in a mold of the desired shape in the temperature range of 180° to 250° C. It is desirable to carry out the polymerization in a stepwise manner in which the polymerizable mixture is heated at 180° C for a period of time and then at 205° C for another period of time. The polymer can then be removed from the mold and further heated (post-cured) at 250° C until all the polymerizable groups are substantially converted. The resulting polymer is typically strong, stiff, and highly resistant to water, organic solvents and heat, and can be used as a structural material. The polymerizable mixture can be advantageously combined with fillers, reinforcements, or continuous fibers known to those skilled in the art before polymerization.

Because the polymers of this invention are substantially insoluble and infusible, identification of their chemical structures is difficult. It is believed, however, that an isomerization of the chromene double bond takes place, follows by polymerization as outlined in the equation below.

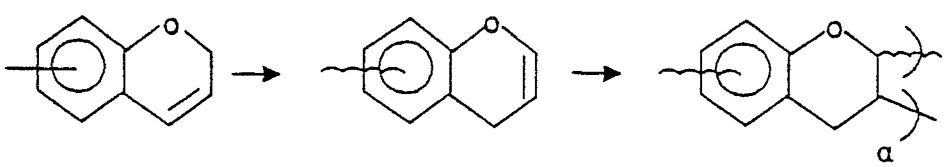

It is, of course, possible that the direct polymerization of the chromene without isomerization and that copolymerization of the isomerized double bond with the unisomerized one can also occur to some, but minor, extent. As the polymerization of the chromene functions takes place, the propargyl ether functions are simultaneously converted to the chromenes.

The invention will now be described with reference to the following examples. Concentrations are percent by weight unless otherwise specified. These examples are exemplary only and are not intended to be limiting.

## Example 1

### Preparation of Aromatic Propargyl Ethers

The following illustrates the general method of preparation of the subject ether reactant. Forty-five point six (45.6) grams of bisphenol A (0.2 moles), 200 milliliters of 20 percent aqueous sodium hydroxide, and 3.22 grams of tetrabutylammonium bromide (0.01 moles) were combined at 20° C. To this mixture at 20° C, 34.27 grams of propargyl chloride (0.46 moles) was added over a 10 minute period, and the mixture was stirred overnight at room temperature for 16 hours. This produces white crystals that were filtered, washed two times with 200 milliliters of water and two times with 50 milliliters of isopropanol. This produced the desired bispropargyl ether. The bispropargyl ether weighed, after drying 57.9 grams, for a yield of 95.2 weight percent. Also it had a melting point of 83° C and a purity, measured by gas chromatography, of 99.7 percent.

This example proceeded according to the following formula:

$$\text{Bisphenol A} + 2.3\ HC \equiv C\text{-}CH_2Cl + NaOH + H_2O$$

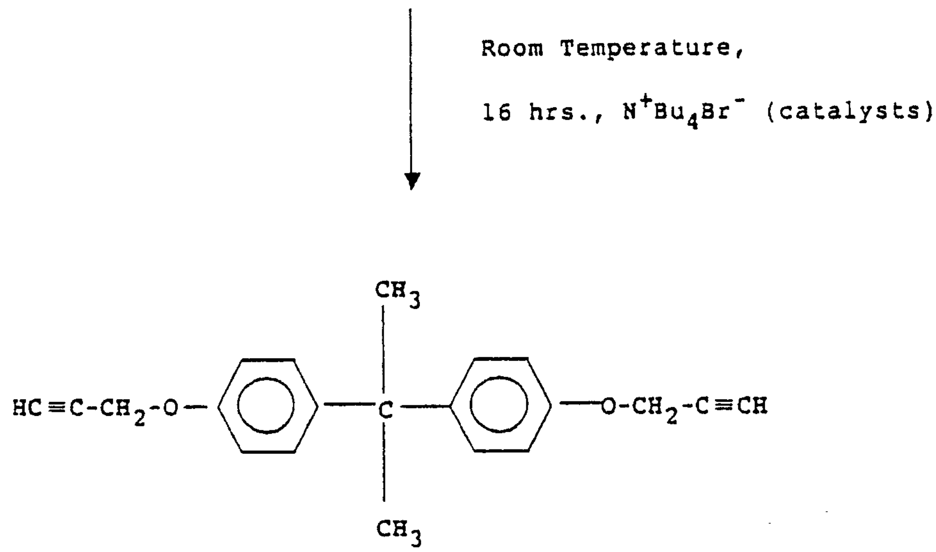


Due to the high purity of the product, no recrystallization was necessary to recover it.

Example 2

Example 1 was repeated. However, the reactants were stirred at 50° C for a period of 4 hours and 2 to 5 mole percent of tetrabutylammonium bromide was used as the phase transfer catalyst. This produced 85 to 97 percent yield of bispropargyl ether having greater than 98 percent purity. Accordingly, no recrystallization was necessary to recover the bispropargyl ether.

Similar experiments can be conducted which give the following propargyl ethers:

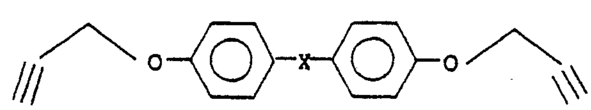

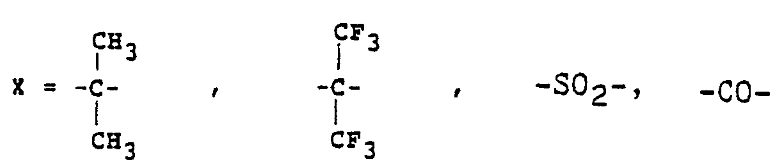

Example 3

Preparation of Compositions Containing Chromenes Derived From The Bispropargyl Ether of Bisphenol A (VI)

A mixture of aromatic propargyl ether VI (500 gms) and 2.20 1 of DOWTHERM J (Trademark of The Dow Chemical Company: mixture of isomers of diethylbenzene) was stirred and heated under gentle reflux with a nitrogen blanket for 42 hours. Analysis of an aliquot by gas chromatography (narrow bore capillary column, 15 m, bonded with DB-5, 0.25 µm) shows 41.4 percent bischromene V, 43.24 percent monochromene-monopropargyl ether IV and 13.9 percent bispropargyl ether VI. Most of the solvent was distilled off under reduced pressure and complete removal of solvent was effected by Wiped Film distillation. The dark red liquid resin (460 gms) containing 63 percent chromene was analyzed by gas chromatography (gc) using an internal standard (1-phenyl decane) and found to have no oligomers. DSC run showed an exotherm of 462 J/g.

Example 4

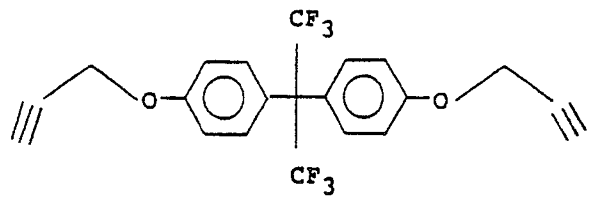

A mixture of the bispropargyl ether of hexafluorobis phenol A (179.4 gms) and 500 ml of 1,2,4-trichlorobenzene was stirred and heated under reflux with nitrogen blanket for 8 hours. Gc analysis of an aliquot showed 44.1 percent monochromene, 37.7 percent bischromene and 15.6 percent bispropargyl ether of hexafluorobisphenol A. Solvent was removed as described before and the dark red resin (179 gms) containing 59.8 percent chromene showed an exotherm of 400 J/g.

Example 5

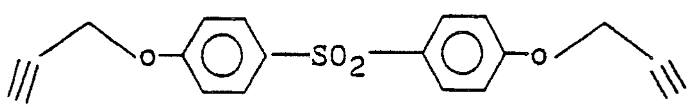

A mixture of the bispropargyl ether of bisphenol S (50 gms) and 150 ml of 1,24-trichlorobenzene was stirred and heated under reflux with a nitrogen blanket for 16 hours. After the removal of solvent, the dark red resin containing 62 percent chromene was used for polymerization.

Example 6

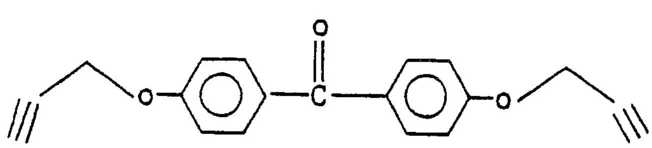

A mixture of the bispropargyl ether of bisphenol K (72 gms) and 220 ml of trichlorobenzene was stirred and heated under reflux with a nitrogen blanket for 14 hours. Removal of solvent provided the dark red resin

containing 60 percent chromene.

Example 7

A mixture of ether (VI) (100 gms) and 320 ml of tetralin was stirred and heated under reflux with a nitrogen blanket for 6 hours. Gc analysis of an aliquot showed 35.3 percent bischromene, 45.2 percent monochromene-monopropargyl ether and 17.2 percent bispropargyl ether. Solvent was removed as described in the previous paragraph and the dark red resin (95 gms) containing 57.9 percent chromene showed an exotherm of 515 J/g and was found to have no oligomers as shown by Gc.

Example 8

Preparation of Compositions Containing Chromenes IV And V From Bispropargyl Ether Catalyzed By Cuprous Chloride

To a stirred mixture of the bispropargyl ether of bisphenol A (833.51 g) and o-dichlorobenzene (2.5 L) under a blanket of nitrogen was added 0.6 ml of a solution of cuprous chloride in concentrated hydrochloric acid (1 g/4 ml). The mixture was stirred and heated under reflux for 5 hours then cooled to room temperature. Several similar runs were performed and the reaction mixtures were filtered through filter paper and combined. Solvent was removed on a rotary evaporator using aspirator vacuum with a bath temperature of 85 °C followed by wiped film distillation with a wall temperature of 105 °C and a vacuum of 0.5 mm Hg. The resultant material had a low viscosity of 46000 cps at 25 °C and 2890 cps at 40 °C versus 49500 cps at 40 °C and 2610 cps at 60 °C for material prepared by the process described in Example 3. Thus, the material is eminently suitable for further formulation with additives followed by molding. Good castings free of voids were obtained from the resin. Analysis of a sample of the resin by gas chromatography showed that there was 42.55 percent bischromene V, 42.53 percent monochromene IV, and 12.48 percent bisether VI. Without catalyst, 36 hours was required to achieve approximately the same conversion.

Thus, the catalyst allows the preparation of low viscosity mixtures containing chromenes IV and V from aromatic propargyl ether VI by drastically reducing the time necessary for the rearrangement. Short reaction times and low viscosity chromene compositions are the advantages of using the catalyst.

Example 9

Conversion of Bispropargyl Ether VI Into Chromenes IV And V Catalyzed By Cupric Bromide

To a stirred mixture of VI (25 g) and 75 ml of o-dichlorobenzene (o-DCB) under $N_2$ was added 25 mg (1000 ppm, 0.1 weight percent) of a cupric bromide and the mixture was stirred and heated under reflux for 4 hours. Gc analysis shows 49 percent of bischromene V, 41 percent of monochromene IV with about 9 percent of the ether VI.

Example 10

Catalysis By Zinc Chloride

To a stirred mixture of VI (110 g) and 350 ml of o-DCB under nitrogen was added 110 mg (1000 ppm) of zinc chloride and the mixture was stirred and heated under reflux for 5 hours. The solvent was removed and the dark red resin (109 g) was recovered. Gc analysis showed the chromene content to be 54 percent.

Example 11

Formation of Chromenes Directly From Bisphenol A

A mixture of bisphenol A (146 g, 0.64 mol) and 400 ml of 5N aqueous sodium hydroxide solution was stirred at ambient temperature for 30 minutes. Tetrabutylammonium bromide (6.44 g, 0.02 mol) and propargyl chloride (92.6 ml, 1.28 mol) were added. The mixture was stirred and heated to 45 °C over 30 minutes. After stirring at 45 °C for 2 hours, an additional amount of propargyl chloride (9.26 ml, 0.128 mol) was added and heating at 45 °C was carried out for another 2 hours. There was added 500 ml of o-DCB and 200 ml water to the mixture. It was stirred for a few minutes. The DCB layer was separated and passed through a column of cation exchange resin (DOWEX MSC-1: Trademark of The Dow Chemical Company) (pre-dried at 70 °C for 16 hours under vacuum, 150 gms, 26 x 3 cm). The column was further washed with 2 x 100 ml of toluene. The combined organic solution was placed in a 2 1 3 neck flask. Forty (40) µl of a solution of cuprous chloride in concentrated hydrochloric acid (1 g/4 ml) (50 ppm) was added. The mixture was heated to reflux with the overhead collection of volatile liquids. The mixture was refluxed at 185 to 186 °C for 4 hours and 50 minutes. GC analysis showed 9 percent of the bisether, about 41 percent of the monochromene and 49 percent of the bischromene. The solvent was removed under reduced pressure. The product containing VI, IV, and V was obtained as a dark red, low-viscosity resin weighing 183.2 g (94 percent). DSC of the resin showed an exotherm of 453 J/g with the maximum at 273.9 °C.

Polymerization of the material prepared according to the in situ process outlined above proceeded smoothly by heating at 200 °C for 2 hours and at 250 °C for 2 hours leading to a substantially void-free molding with little or no shrinkage.

Example 12

Polymerization Of Chromene Containing Mixtures

A mixture containing chromenes prepared as described above in Example 3 was placed in a vacuum oven maintained at 180 °C. Vacuum (0.1 mm Hg) was applied and the material was degassed for about 60 minutes. The degassed chromene mixture was then carefully poured into preheated (approximately 170 °C) stainless steel parallel plate molds which were pretreated with TEFLON (Trademark of E. I. du Pont de Nemours & Company) mold release agent. The mold was then placed in an oven and cured at 205 °C for 2 hours and 250 °C for 2 hours. The plaque was then removed. A free standing post-cure at 275 °C for 2 hours was used to increase the glass transition temperature.

Alternatively, triethylenetetraamine (TETA) was added to the degassed chromene mixture at a 1 weight percent level prior to molding. This improved the moldability of the material.

Example 13

Evaluation Of Polymerized Chromene Mixture

A mixture containing chromenes was degassed at 185 °C for 75 minutes. Triethylenetetraamine (1 weight percent) was added and the sample was mixed thoroughly. The material was poured into a preheated (205 °C) parallel plate mold and cured at 205 °C for 2 hours and 250 °C for 2 hours. The mold was opened and a plaque was obtained. The material was evaluated for mechanical, physical, and electrical properties as shown below.

| Properties of Material as Cured* | | |
|---|---|---|
| Property | | |
| Dielectric Constant 10 kHz, dry | 2.93 | |
| % Moisture Uptake Boiling Water, 330 h | 1.7 | |
| Glass Transition Temperature (TMA) | 255° C | |
| Ultimate Glass Transition Temperature (DMA) | 350° C | |
| Extrap. Onset of Decomposition (TGA) (nitrogen/air) | 397/405° C | |
| Flextural Modulus/ksi | Room Temp. | 635 |
| | 149° C (300° F) | 486 |
| | 204° C (400° F) | 397 |
| Flexural Strength/ksi | Room Temp. | 15.7 |
| | 149° C (300° F) | 13.1 |
| | 204° C (400° F) | 11.7 |

* The chromene mixture used to prepare the casting was prepared by heating the bispropargyl ether of bisphenol A to reflux in trichlorobenzene for 135 minutes followed by removal of solvent.

While flexural modulus and flexural strength vary with curing schedules these properties show unexpected improvement over properties of many state of the art thermoset resins.

Although the invention has been described in conjunction with specific embodiments, it is evident that many alternatives and variations will be apparent to those skilled in the art in light of the foregoing description For example, a person of ordinary skill in the art can use conventional processing conditions for any of the processes described in this disclosure. Accordingly, the invention is intended to embrace all of the alternatives and variations that fall within the spirit and scope of the appended claims.

## Claims

1. A process for the preparation of chromene containing mixtures comprising:
reacting a material containing propargyl ether groups in a solvent at reaction temperature; the material containing the propargyl ether groups being represented by the following formula I:

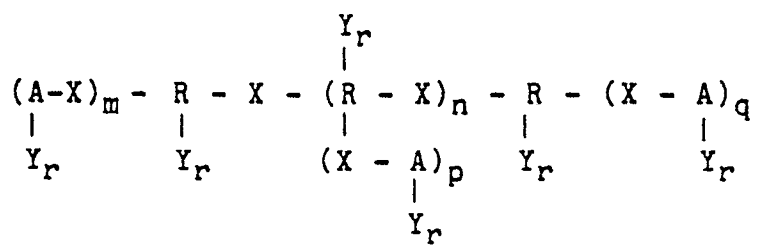

I

where n is an integer from 0 to 200; m, p, q are integers independently from 0 to 6; m + p + q is greater than or equal to 2; r is an integer independently from 0 to 10; A is a $C_6$-$C_{20}$ aromatic radical and bearing at least one propargyl ether group provided that at least one of the positions ortho to the propargyl ether group be unsubstituted; R is a hydrocarbyl radical having from 0 to 50 carbon atoms; Y is halogen
-OAr, -OR′, -SR′, -$SO_2$Ar, -$SO_2$R′, -CO-R′, -CO-Ar,
$-O-\overset{O}{\overset{\|}{C}}-R'$, $-O-\overset{O}{\overset{\|}{C}}-Ar$, $NO_2$, $-NH\overset{O}{\overset{\|}{C}}R'$, $-NH\overset{O}{\overset{\|}{C}}Ar$,
$-\overset{O}{\overset{\|}{C}}-NHR'$, $-\overset{O}{\overset{\|}{C}}-NHAr$, -$SiR'_3$, -Si(OR′)$_3$, R′, H, -OH, or -$CO_2$R′;
R′ is a hydrocarbyl radical having from 0 to 20 carbon atoms; X is absent or is:

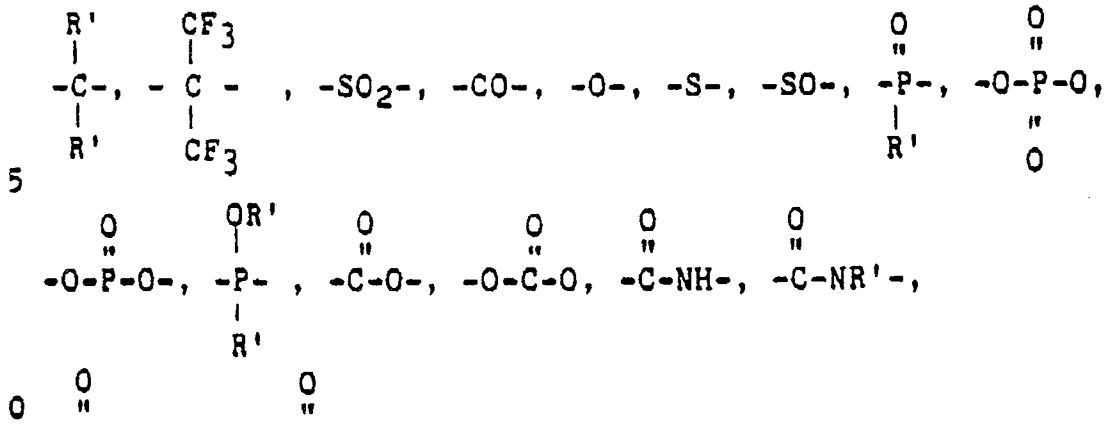

and

Ar is an aromatic radical;
and recovering a chromene containing mixture having the following formula II:

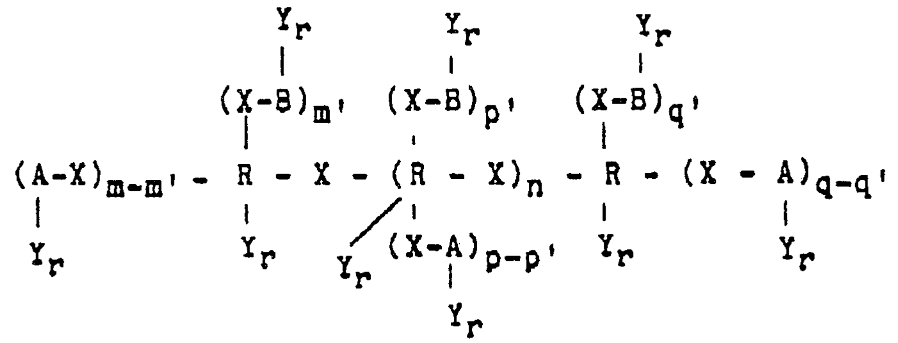

II

and where m′, p′, q′ are integers from 0 to 6; m′ + p′ + q′ is greater than or equal to 0; B is a $C_6$-$C_{20}$ aromatic radical containing at least one chromene group, and where R, Y, X, n, m, p, q, r, A have the representations recited above.

2. The process according to Claim 1, wherein the solvent is diethylbenzene, diisopropylbenzene, dichlorobenzene, trichlorobenzene or tetralin.

3. The process according to Claim 1, wherein the reaction time is between 2 and 12 hours.

4. The process according to Claim 1, wherein the reaction temperature is between 100°C and 200°C.

5. The process according to Claim 1, further comprising a catalyst of a copper salt or a zinc salt.

6. An insitu process for the formation of chromene containing mixtures comprising:
reacting a phenolic compound with propargyl halide in an aqueous solution of an alkaline agent in the presence of a phase transfer catalyst under reaction conditions sufficient to produce a propargyl ether;
adding an inert organic solvent to the propargyl ether:
purifying the solubilized propargyl ether;
reacting the propargyl ether in a suitable solvent at reaction temperature; the material containing the propargyl groups is represented by the following formula I:

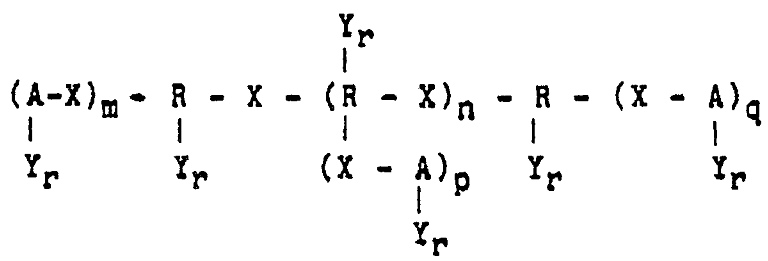

I

where n is an integer from 0 to 200; m, p, q are integers independently from 0 to 6; m + p + q is greater than or equal to 2; r is an integer independently from 0 to 10; A is a $C_6$-$C_{20}$ aromatic radical and bearing at least one propargyl ether group provided that at least one of the positions ortho to the propargyl ether group be unsubstituted; R is a hydrocarbyl radical having from 0 to 50 carbon atoms; Y is halogen

-OAr, -OR′, -SR′, $-SO_2Ar$, $-SO_2R'$, -CO-R′, -CO-Ar,

$-O-\overset{O}{\overset{\|}{C}}-R'$, $-O-\overset{O}{\overset{\|}{C}}-Ar$, $NO_2$, $-NH\overset{O}{\overset{\|}{C}}R'$, $-NH\overset{O}{\overset{\|}{C}}Ar$,

$-\overset{O}{\overset{\|}{C}}-NHR'$, $-\overset{O}{\overset{\|}{C}}-NHAr$, $-SiR'_3$, $-Si(OR')_3$, R′, H, -OH, or $-CO_2R'$;

R′ is a hydrocarbyl radical having from 0 to 20 carbon atoms; X is absent or is:

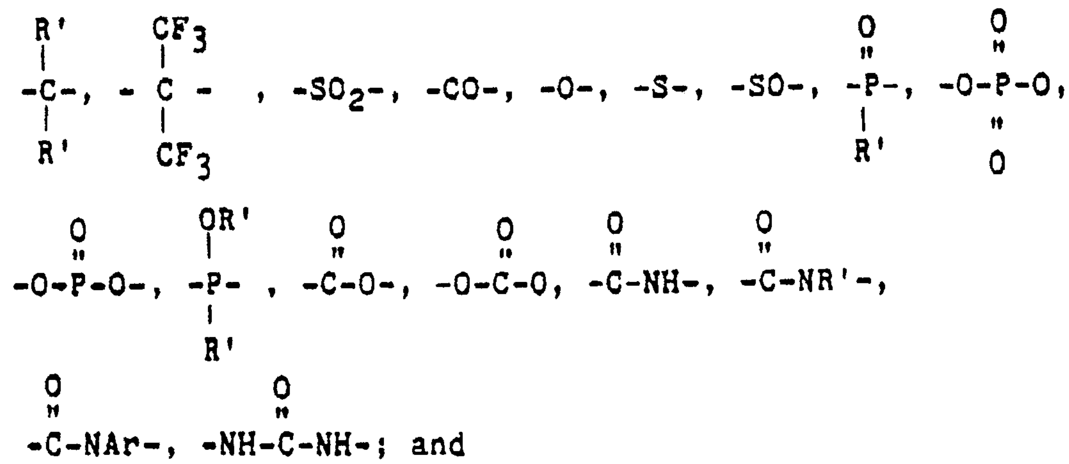

and

Ar is an aromatic radical; and recovering a chromene containing mixture having the following formula II:

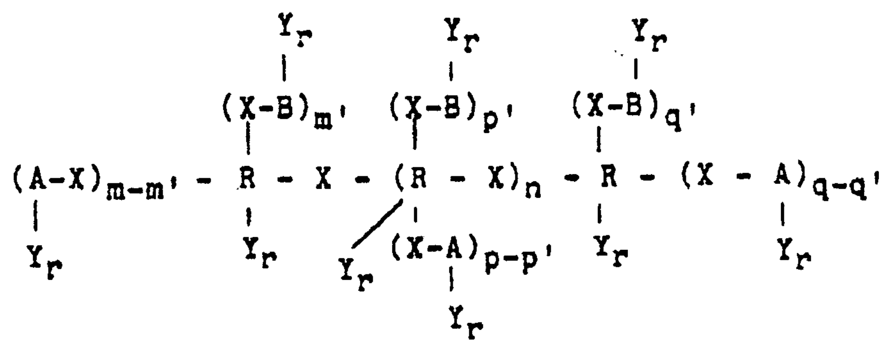

II

where m′, p′, q′ are integers from 0 to 6; m′ + p′ + q′ is greater than or equal to 0; B is a $C_6$-$C_{20}$ aromatic radical containing at least one chromene group; and where R, Y, X, n, m, p, q, r, A have the representations recited above.

7. The process of Claim 6, wherein the propargyl halide has the following formula C: $HC \equiv C-CH_2X$

C

wherein X is Cl, Br, or I, and the phase transfer catalyst is a quaternary salt represented by the following formula D:

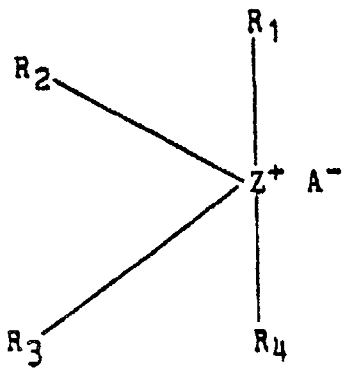

D

wherein Z is a tetravalent ammonium or phosphonium ion, A is a suitable counterion, and each $R_1$, $R_2$, $R_3$ and $R_4$ is independently an alkyl, aromatic or alkyl aromatic moiety containing from 1 to 50 carbon atoms, polyethylene glycol ethers having a molecular weight from 200 to 50,000, crown ethers, or cryptates.

8. The process of Claim 7, wherein the phenolic compound has the following formula A:

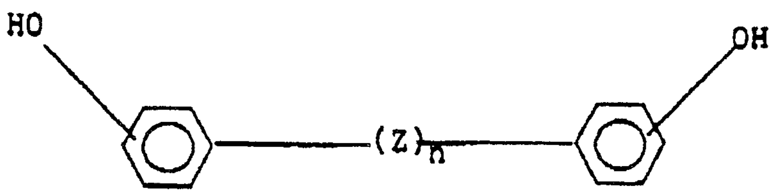

A

where Z is CO, SO, $SO_2$, O, S, $C(CF_3)_2$ or $C(CH_3)_2$ and n is 0 or 1.

9. The process of Claim 7, wherein the phenolic compound has the following formula B:

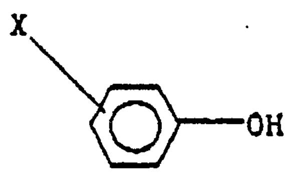

B

where X is 4-phenoxy, 3-methyl or 4-methyl group.

10. A chromene containing mixture comprising the compound of formula I of Claim 1.

11. A process for the polymerization of chromene containing mixtures comprising polymerizing the compound of formula II of Claim 1.

12. A polymer comprising the compound of formula II of Claim 1.

13. An electrical laminate comprising the compound of formula II of Claim 1.